# EUROPEAN PATENT APPLICATION

(11) **EP 4 635 542 A1**
(43) Date of publication of application: **22.10.2025**
(21) Application number: 24170302.4
(22) Date of filing: 15.04.2024
(51) Int. Cl.: A61M 16/06

(54) **IMPROVED HEADGEAR FOR A RESPIRATORY NASAL MASK**

(71) Applicant: Air Liquide Medical Systems, 92182 Antony Cedex (FR)
(72) Inventor: ALBERICI, Luca, 25073 Bovezzo (IT); ROMAIOLI, Roberto, 25073 Bovezzo (IT); RUOCCO, Alessandra, 25073 Bovezzo (IT)
(74) Representative: Air Liquide

(57) **Abstract**

The invention concerns a headgear structure (30) for a respiratory mask (1), comprising several flexible elongated strap elements (31) comprising two lateral strap elements (32) each comprising a proximal end (32a) comprising fixation means (36) configured to be attachable to headgear-connecting structures (15) of the respiratory mask (1), and further a distal end (32b), characterized in that it further comprises an annular-shape strap structure (37) comprising a first curved strap element (33) and a second curved strap element (34), wherein the distal ends (32b) of the two lateral strap elements (32) are firmly attached to the annular-shape strap structure (37). Respiratory nasal mask (1) comprising such a headgear (30).

## Description

The invention concerns a headgear and a respiratory nasal mask comprising a mask body, a flexible nasal cushion detachably coupled or fixed together, and such a headgear detachably attached to the mask body, that is useable in the treatment of respiratory conditions or diseases affecting adults, children, toddlers, infants or newborns, such as obstructive sleep apnea (OSA).

Respiratory masks are commonly used for delivering a flow of breathable gas, such as air under pressure, for, or to assist in, patient respiration. A mask assembly typically comprises a rigid or semi-rigid hollow shell or body, usually made of polymer, and further comprising a soft face-contacting cushion, commonly called a flexible cushion, that comes into contact with the patient's face and conforms to the various facial contours of the patient face for receiving the patient's nose and/or mouth so that the patient can inhale the gas comprised into a breathing chamber that is defined by the hollow shell or body and/or the flexible cushion. The flexible cushion is usually made of soft, resilient elastomeric material, such as soft silicone or similar material.

A gas supply line delivers a respiratory gas, such as air under pressure, to the mask, namely the breathing chamber. An example of such a mask is given by EP-A-462701.

Masks receiving only the patient's nose are called a nasal mask, whereas those receiving the patient's nose and mouth are called facial or oro-nasal masks. Further, masks contacting only the outer regions located around the nostrils of the user are called minimal contact masks (MCM). An example is disclosed by EP-A-4279108.

Further, a headgear, comprising flexible straps or similar structures, is usually attached to the mask body for correctly positioning, holding and/or securing the mask on the head of a patient. The headgear can be adjusted to pull the mask against the face with sufficient force to achieve a gas tight seal between the mask and the wearer's face as taught by EP-A-462701, EP-A-874667, EP-A-1972357, EP-A-3981455 or WO-A-00/57942. Other types of masks comprising a front shroud adapted to attach the headgear are disclosed by EP-A-2708258 or US-A-2007/0044804.

Generally speaking, masks can be used for treating various respiratory conditions or diseases affecting adults or children, including some toddlers, infants or newborns, such as obstructive sleep apnea (OSA) or other pathologies. In particular, the effective treatment of OSA requires that the patient uses the nasal mask overnight, namely when he/she sleeps, which leads to various problems.

Despite the fact that many different architectures of masks have been proposed so far, especially for treating OSA or the like, problems or drawbacks still exist with existing nasal masks.

One of the main problems that still exist is related to the discomfort of use for the patient. Various causes of discomfort exist, such as gas leaks due to a wrong assembling of the mask components, a poor positioning of the mask of the patient's face, poor tightness of the cushion, especially overnight...

Some causes of discomfort are related to the headgear that does not ensure a good tightness due to loose settings and/or to a poor design of said headgear.

A goal of the present invention is to provide an improved headgear for a minimal contact mask (MCM) that ensures a sufficient force to achieve a gas tight seal between the mask and the wearer's face, an efficient positioning and securing of the mask on the patient's face, thereby limiting or preventing the escape of gas, i.e. gas leaks, when donned by a user, including overnight.

Preferably, the improved headgear according to the present invention should also be simple to attached to or detached from the mask body, light and comfortable to wear and in use for the patients, when donned, for instance for treating OSA or other pathologies requiring a gas delivery by mask.

A solution according to the present invention concerns a headgear structure, i.e. also called a headgear, for a respiratory mask, preferably a nasal mask, such as a MCM, comprising several flexible elongated strap elements comprising two lateral strap elements each comprising a proximal end comprising fixation means configured to be attachable to headgear-connecting structures of the respiratory mask, and further a distal end.

Further, the headgear structure further comprises an annular-shape strap structure comprising a first curved strap element and a second curved strap element, wherein the distal ends of the two lateral strap elements are firmly attached to the annular-shape strap structure.

The headgear according to the present invention can further comprise one or more of the following features.
- the annular-shape strap structure has an elongated shape with a width (W) and a height (H) so that : W ≥ 2 . H.
- the width (W) of the annular-shape strap structure is of between 22 cm and 35 cm, preferably of between 24 cm and 30 cm, for instance of between 26,5 and 28,5 cm.
- the height (H) of the annular-shape strap structure is of between 7 cm and 11 cm, preferably of between 7,5 cm and 9,5 cm, for instance of about 8,4 cm.
- the lateral strap elements have a first width (W1) of between 1 cm and 2 cm, preferably of between 1,1 cm and 1,5 cm, for instance of about 1,2 cm.
- the straps of the annular-shape strap structure have a second width (W2) of between 1 cm and 2 cm, preferably of between 1,1 cm and 1,5 cm, for instance of about 1,2 cm.
- according to one embodiment, W1=W2.
- the annular-shape strap structure comprises two opposite end regions.
- the distal ends of the two lateral strap elements are attached to the opposite end regions of the annular-shape strap structure.
- the two opposite end regions of the annular-shape strap structure are diametrally-opposite.
- preferably, the distal ends of the two lateral strap elements are attached to the opposite end regions of the annular-shape strap structure, preferably by Radio Frequency (RF) welding or any other suitable process/method.
- the opposite end regions of the annular-shape strap structure comprise each an enlarged zone.
- the distal ends of the two lateral strap elements are attached to said enlarged zones or portions of the opposite end regions of the annular-shape strap structure.
- each enlarged zone or portion has a third width (W3) of between 1,5 cm and 3 cm, with W3 > W2, for instance of between 1,8 and 2,4 cm.
- the enlarged zones are arranged on the first curved strap element.
- the lateral strap elements and the first and second curved strap elements can have a thickness of several millimeters.
- the first curved strap element forms an upper or top half of the annular-shape strap structure.
- the second curved strap element forms an lower or bottom half of the annular-shape strap structure.
- the first curved strap element is curved upwardly or bent, i.e. toward the top of the head of the user, when donned.
- the second curved strap element is curved or bent downwardly, i.e. toward the neck of the head of the user, when donned.
- the annular-shape strap structure has a generally oval-shape, i.e. oval, ellipsoidal or similar.
- the annular-shape strap structure is flexible.
- the two lateral strap elements are flexible.
- two lateral strap elements and the annular-shape strap structure have a multi-layer structure, preferably a 3-layer structure.
- the multi-layer structure comprises several superimposed layers that are made integral, i.e. that are firmly fixed or bonded together.
- the multi-layer structure comprises a first fabric layer comprising at least a first polymer material.
- the multi-layer structure comprises at least a second fabric layer comprising at least a second polymer material.
- the multi-layer structure comprises at least an intermediate foam layer sandwiched between the first fabric layer and the second fabric layer.
- the first fabric layer, the intermediate foam layer and the second fabric layer are superimposed.
- the first fabric layer comprises a fluffy surface/coating that provides an smooth feeling to the patient, when donned, i.e. when in contact with the patient's skin and/or head.
- the second fabric layer comprises a soft surface/coating.
- the first polymer material and/or the second polymer material are chosen among polyamide materials, such as Nylon ^{®}.
- the intermediate foam layer comprises polyurethane material
- the first fabric layer, the second fabric layer and the intermediate foam.
- the layer are bonded, i.e. fixed, together by flame lamination.
- the opposite edges of the multi-layer structure comprising the first fabric layer, the second fabric layer and the intermediate foam layer are sealed, preferably by flame lamination.
- the fixation means arranged at the proximal ends of one or of the two lateral strap elements comprise Velcro^{®}-type structures.
- the Velcro^{®}-like structures are configured for being foldable for forming loops or the like, thereby allowing attaching the headgear to the headgear connecting structures of the mask body.
- the/each Velcro^{®}-like structure comprises a little-hook region configured for being attached to a little-loop region arranged on one or each lateral strap element(s), when the free proximal end is folded over, thereby forming a fixation loop.
- at least one of the two lateral strap elements can comprise markings arranged on the outer surface of said lateral strap element(s), representing different tightening levels or forces of the straps.
- the lateral strap elements and the first and second curved strap elements have a band or ribbon shape.
- the lateral strap elements and the first and second curved strap elements are stretchable, i.e. elastic, and flexible.
- the headgear is configured for, when used/donned, positioning, maintaining and/or securing a nasal mask in a desired position on the patient's nose regions.
- the headgear constitutes a tridimensional structure that is conceived for being installed on the head of the user.
- the annular-shape strap structure constitutes a central part of the headgear, i.e. the annular-shape strap structure is situated between the two lateral strap elements.
- the lateral strap elements projects from the central annular-shape strap structure, i.e. they ara diametrally-fixed to the central annular-shape strap structure.
- the lateral strap elements comprise a right strap element and a left strap element.
- the axis XX of the first curved strap element and the axis YY of the second curved strap element define together, in the two opposite end regions, an angle B of less than 90°, preferably of less than 80°, more preferably of less than 70°, typically of about 40° to 60°, for instance of between 45° and 50°.

The invention further concerns a respiratory nasal mask, preferably a "minimal contact mask" (MCM), comprising:
- a mask body comprising a front opening and two elongated arms attached to opposite lateral sides of the mask body, i.e. left and right sides, said elongated arms comprising headgear-connecting structures,
- a hollow flexible cushion comprising a supple rear part comprising a rear breathing opening for allowing, in use, gas exchanges with nostrils of a user, i.e. a patient, said hollow flexible cushion being detachably attached to the mask body, and
- a headgear structure according to the invention, i.e. as above described, attached to at least one of the headgear-connecting structures of the two elongated arms, preferably to both headgear-connecting structures.

The respiratory mask according to the present invention can further comprise one or more of the following features:
- the hollow flexible cushion comprises a supple rear part comprising peripheral edge regions surrounding the rear breathing opening that are configured to, in use, come into contact and provide a gas sealing with areas located immediately around the nostril edges of the user.
- the two lateral arms project, one on each lateral side of the mask body, toward the cushion so that the cushion is sandwiched between said two lateral arms.
- the two lateral arms are made one-piece with the mask body.
- the mask body is made of polymer material.
- the supple rear part of the flexible cushion comprises an upper lip area (ULA) located immediately under the nostrils of the patient (P), two curved alae (CA) on both sides of the nostrils of the patient (P) and a tip region (TR) of the nose of the patient (P), when the mask (1) is donned by the patient (P).
- the cushion is made of silicone.
- a tubular hollow connector is secured to the front opening of the mask body, preferably rotatably secured to the front opening.
- the headgear-connecting structures of the two lateral arms comprise slots, holes, hooks or the like.
- the two lateral arms and the mask body are made of a rigid or semi-rigid polymer material.
- the rigid or semi-rigid polymer material, i.e. a plastic material, is polycarbonate (PC), polypropylene (PP), Nylon^{®}, or the like, for instance PC.
- the two lateral arms have an elongated shape, such as a band or ribbon shape. Other shapes are of course also possible.
- the lateral arms have each a length of about 3 to 10 cm, preferably of about 3 to 6 cm.
- each lateral arm comprise a free end comprising the headgear-connecting structures for attaching a headgear thereto.
- the mask body comprises a front wall traversed by the front opening, preferably a curved front wall.
- the mask body comprises two opposite surfaces, namely a front surface (i.e. toward the outside) and a rear surface facing the cushion (i.e. toward the inside).
- the front wall has a length (L) of between 40 and 70 mm and a width (W) of between 30 and 50 mm.
- the front wall has a thickness of between 2 and 15 mm.
- the front wall is flat.
- the front wall is slightly foldable, i.e. can be slightly bent, especially when tension forces are applied by the headgear connected to the two arms.
- the front wall comprises two opposite tips or sides, i.e. right and left sides, the two lateral arms being attached to said two opposite tips or sides.
- the front opening is arranged at the center of the front wall, namely constitutes a central opening.
- the front opening has a circular shape, i.e. a circular section.
- the front opening has a diameter of between 15 and 25 mm, preferably of between 17 and 22 mm.
- the front wall of the mask body further comprises several venting orifices, i.e. holes, slots or similar, traversing the front wall and arranged around the front opening.
- the venting orifices are in fluid communication with the inner breathing chamber of the flexible nasal cushion.
- the venting orifices are arranged in groups or arrays comprising each several venting orifices.
- groups or arrays of venting orifices are arranged so as to sandwich the front opening of the mask body, i.e. arranged to the left and to right of the front opening.
- the venting orifices traverse the front wall and open on the rear surface of the mask body between the collar structure and the tubular rear section of the mask body.
- the venting orifices are configured for venting to the atmosphere at least a part of the CO₂-containing gaseous flow expired by the user during the expiratory phases.
- the venting orifices have a cylindrical or tronconical shape, preferably their section is greater on the rear surface of the mask body than on the front surface of said mask body.
- the flexible cushion comprises an inner breathing chamber, i.e. an inner room or compartment for the gases, i.e. gas delivered to the user, such as a patient, during inspiratory phases and/or CO₂-enriched gases exhaled by the user during expiratory phases.
- the cushion is sized and designed for not covering the regions of the nose of the user situated above his/her nose tip or tip region (TR), when the mask is worn by said user, especially the dorsum, i.e. cartilaginous and/or bony dorsum.
- the cushion is made of a flexible material, i.e. a supple and soft or semi-soft material, such as silicone or the like.
- the rear opening is in fluid communication with the inner breathing chamber.
- the front opening of the mask body is in fluid communication with the inner breathing chamber of the flexible cushion
- the rear opening of the cushion has (almost or quasi) rectangular or trapezoidal shape.
- the peripheral edge regions surrounding the rear opening of the flexible cushion comprise a peripheral thin wall, such as a membrane or the like, constituting a sealing, preferably a supple sealing skirt, around said rear opening so as to ensure an efficient gas tightness while in contact with the user's face, when the latter wears the mask, preferably all along the outer periphery of said rear opening.
- the peripheral thin wall of the peripheral edge regions of the cushion forming the sealing comes into contact and provide a gas sealing with the areas located immediately around the nostril edges of the use, namely the upper lip area (ULA), the two curved alae (CA), and the tip region (TR) of the nose.
- the rear surface of the flexible cushion is curved, in particular for matching, in use, the two curved alae (CA) of the nose of the user.
- the peripheral edge regions surrounding the rear opening of the flexible nasal cushion form a curved (quasi) contacting surface, preferably a triangular or trapezoidal curved contacting surface.
- the curved (quasi) contacting surface surrounding the rear opening comes into contact, in use, i.e. when the user wears the mask, with the areas (i.e. user's facial areas) located immediately around the nostril edges of the user thereby providing an efficient gas sealing.
- the peripheral edge regions of the rear opening of the flexible cushion comprises a base border, two lateral panels or wings, and a top border.
- the width of the top border is larger/greater than the width of the base border, for instance the width of the top border is between 17 and 25 mm, whereas the width of the base border is between 10 and 20 mm.
- the peripheral thin wall forming the sealing skirt around the rear breathing opening is molded in one piece with the rest of the cushion.
- a bridge element or support-element is arranged in the rear opening.
- the bridge element has an arch-shape or the like that projects into the cushion.
- the bridge element has a "U" or "V"-shape or similar.
- the bridge element forms a bridge between the upper and lower edges of the rear opening.
- when the mask is worn, the bridge element is in contact with the outer region of the nose of the patient separating the nostrils so as to constitute a support or the like prohibiting a collapse, sagging or similar of the thin regions of the cushion, namely the peripheral edge regions of the rear opening of the flexible cushion, in particular the base and top borders.
- the peripheral edge regions of the rear opening of the flexible cushion are thinner than the tubular front part regions of the flexible cushion.
- the thickness of the wall of the flexible cushion in the peripheral edge regions of the rear opening is less than 1 mm, preferably of between 0.30 and 0.6 mm.
- the flexibility of the cushion is greater in the peripheral edge regions of the rear opening than in the tubular front part regions of said flexible cushion.
- the mask body and the cushion comprise connecting structures, i.e. fastening means, for attaching the cushion directly to the mask body, in a detachable manner so that the user can disassemble them for cleaning operations for instance.
- the fastening means comprise an annular shoulder or flange arranged in the mask body cooperating with an annular groove arranged in the flexible cushion.
- the annular shoulder or flange of the mask body is lodged and retained into the annular groove of the flexible cushion, when the hollow flexible cushion is attached to the mask body.
- a flexible hose is connected to the front opening of the mask body by means of a hollow connector.
- the hollow connector is a straight connector, i.e. tubular shape, preferably having a cylindrical shape.
- the hollow connector comprises an inner passage for the gas, i.e. a lumen.
- the hollow connector is inserted into, i.e. plugged, and held in position in the mask body, through the front opening of the mask body.
- the hollow connector is detachably fixed to the mask body.
- the hollow connector is rotatable with respect to the mask body.
- the hollow connector is configured for connecting a flexible hose thereto, i.e. a gas line or the like. In other words, the tubular connector is adapted for receiving a flexible conduct or hose that feeds a respiratory gas under pressure to the respiratory mask. The gas is fed into the flexible gas conduct or hose by a gas source, such as a medical ventilator, a medical gas-delivery device or the like.
- the hollow connector is in fluid communication with the breathing chamber of the cushion thereby allowing gas to circulate from the hollow connector to the cushion or in the opposite way.
- the hollow connector is inserted and firmly hold into the front opening of the mask body, i.e. secured therein, for instance by a snap-fit connection, a press-fit connection or others
- the hollow connector has a cylindrical or tronconical shape.
- according to another embodiment, the connector is a hollow curved or elbow connector, i.e. having a "L-shape" or the like.
- the connector is made of polymer material, e.g. plastic.

The invention also concerns a respiratory assembly or installation for providing gas to a patient (P) comprising a gas delivery device, such as a medical ventilator, i.e. a respiratory assistance device or the like, a nasal mask, and a headgear attached to the mask according to the present invention, wherein the gas delivery device is connected to the nasal mask by means of the flexible hose.

Such a respiratory assembly comprising a nasal mask according to the present invention is usable in the treatment of respiratory conditions or diseases affecting adults, children, toddlers, infants or newborns, such as obstructive sleep apnea (OSA).

A non-limitative embodiment of a headgear for a respiratory mask, typically a nasal mask, in particular a MCM-type mask, according to the present invention is shown in the enclosed Figures, among which:
- Figure 1 shows an embodiment of a nasal mask usable with a headgear according to the present invention.
- Figure 2 shows the nasal mask of Fig. 1, when equipped with a headgear according to the prior art, when donned by a patient.
- Figure 3 shows the nasal mask of Fig. 1, when equipped with a headgear according to the present invention, when donned by a patient.
- Figure 4 shows an embodiment of a headgear according to the present invention.
- Figure 5 is a scheme of the annular part of the headgear of Fig. 4.
- Figure 6 shows the multi-layer structure of the headgear of Fig. 4.

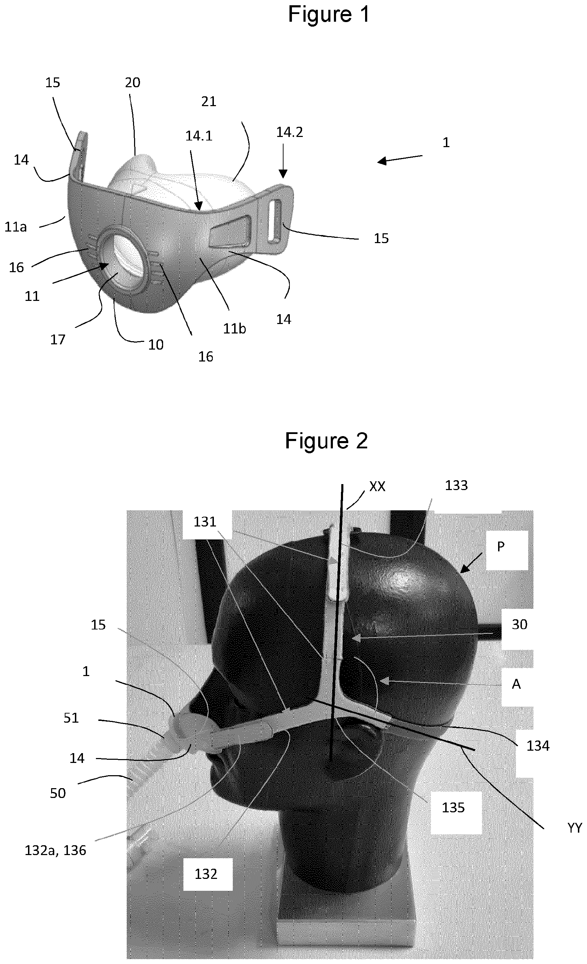

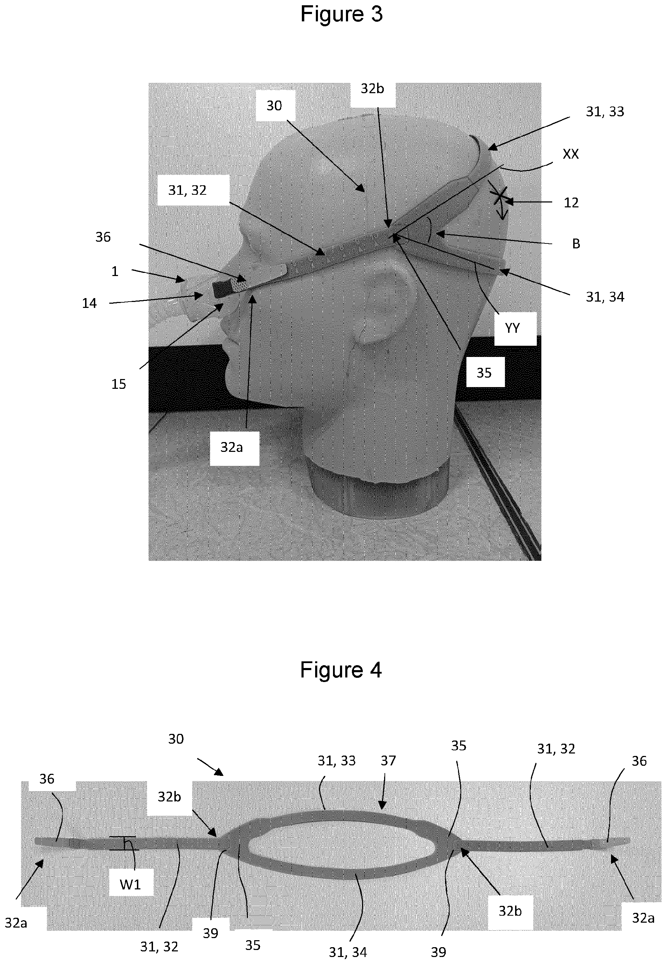

Figure 1 shows an embodiment of a respiratory nasal mask 1, also called a minimal contact mask (MCM), useable with a headgear structure 30, i.e. also called a headgear, according to the present invention, as shown in Fig.3-6.

The nasal mask 1 of Fig. 1 comprises two main parts assembled or coupled together, namely a mask body 10 and a flexible cushion 20 attached together in a detachable manner so that they can be disassembled for instance for cleaning operations or for replacing a worn out cushion 20, and easily reassembled afterwards. The mask 1 has a simple and light structure, and further facilitates the assembling/disassembling of the different components of the mask 1 by the users, namely the patients, especially during daily cleaning operations. Such a mask 1 further exhibits a good comfort as it is lighter and has a low risk of gas leaks.

As shown in Fig. 2 & 3, a respiratory gas, such as air, is provided by a flexible hose 50 fluidly and mechanically connected to the mask body 10 by means of a tubular connector 51 arranged at the proximal end of said flexible hose 50. The distal end of the flexible hose 50 comprises an additional tubular connector for fluidly and mechanically connecting the flexible hose 50 to a medical ventilator (not shown) or a similar apparatus that provides the respiratory gas to the flexible hose 50.

Further, the mask body 10 of the nasal mask 1 comprises comprising two lateral arms 14 attached to opposite lateral sides 11a, 11b of the mask body 10, namely to the left side 11a and to the right side 11b as shown in Fig. 1, namely a right arm and a left arm. The two lateral arms 14 further project toward the rear side of the mask 1, i.e. so as to "sandwich" the cushion 20 as shown in Fig. 1.

A front opening 11 traverses the mask body 10, preferably the front opening 11 is arranged at the center of the mask body 10. The front opening 11 comprises a rim or outer border having a circular section or the like.

The two lateral arms 14 are arranged in a symmetrical manner with respect to the mask body 10. They have elongated shapes, such as band or ribbon shapes.

According to some embodiments, the two lateral arms 14 can be slightly incurved to better match, if desired, some of the contours of the cushion 20.

Each lateral arm 14 can have for instance a length of about 3 to 10 cm, preferably of about 3 to 6 cm.

Furthermore, the lateral arms 14 comprise a free end 14.2 equipped with headgear connecting structures 15, such one or several slots, holes, hooks or the like, as shown in Fig. 1. These headgear-connecting structures 15 are designed for attaching a headgear structure 30 thereto as shown in Fig. 2 & 3, as below detailed.

Further, as shown in Fig. 1, the front opening 11 of the mask body 10 constitutes the entry of an inner passage, i.e. a lumen or the like, of a conduit 17 in fluid communication with the inner chamber of the cushion 20. The conduit 17 projects away or protrudes from the rear surface or side of the mask body 10, i.e. toward the cushion 20.

Fastening means arranged on the rear surface or side of the mask body 10 and on the cushion 20 are used for firmly securing the cushion 20 to the mask body 10. As taught by EP-A-427910, said fastening means can comprise at least a portion of the conduit 17 comprising an annular shoulder or flange, that cooperates with an annular groove (not shown) arranged in a front opening (not shown) of the cushion 20, preferably the annular shoulder is lodged in the annular groove, for securing the cushion 20 to the mask body 10. Preferably, a collar structure coaxially arranged around the conduit 17, on the rear surface of the mask body 10, can be provided for improving the attachment of the cushion 20 to the mask body 10. Of course, other types of fastening means can be used.

Generally speaking, the flexible cushion 20 is detachably fixed to the mask body 10 for facilitating a replacement or a cleaning of the cushion and/or of the mask body 10.

Further, the hollow flexible cushion 20 comprises an inner chamber for the gas and a supple rear part 21 comprising a rear breathing opening for allowing, in use, gas exchanges with the two nostrils of a user P as below detailed. The inner chamber is located between the front opening and the rear opening of the cushion 20 thereby allowing a fluid communication from the front opening to the rear opening, via the inner chamber, typically during inspiration phases, and vice versa, typically during expiration phases. The flexible cushion 20 constitutes a tridimensional hollow flexible structure defining the inner breathing chamber.

As the mask is preferably a MCM, the supple rear part 21 of the flexible cushion 20 comprises peripheral edge regions surrounding the rear breathing opening that are configured to, in use, come into contact and provide a gas sealing with areas located immediately around the nostril edges of the user P comprising an upper lip area (ULA) located immediately under the nostrils, two curved alae (CA) on both sides of the nostrils and a tip region (TR) of the nose, thereby providing a minimal contact surface or the like with facial areas of the user. In other words, the regions located immediately around the rear breathing opening of the cushion 20 constitute contact zones contacting said specific nasal areas of the user.

The cushion 20 of the nasal mask 1 of the present invention has a compact size and does not extend over the top of the nose, namely does not cover the dorsum regions, i.e. cartilaginous and/or bony dorsum, when the mask 1 is worn by a user P as shown in Fig. 2 & 3, which reduces and/or minimizes the contact with the skin of the user as it rests only on the areas located immediately around the entrances of the nostrils of said user. In other words, the compact cushion 20 can be sized and/or designed to take into account only the width of the bottom of the nose of the user.

Preferably, in order to provide efficient gas tightness (i.e. seal) and/or good comfort for the patient, the cushion wall in the peripheral edge regions surrounding the rear opening of the supple rear part 21 of the cushion 20 is preferably designed or conceived as a thin membrane, i.e. a soft flexible membrane, that comes into contact with the patient's facial areas or regions, namely the upper lip area (ULA), the two curved alae (CA) and tip region (TR) as above stated, so as to better match his/her facial morphology. Said flexible thin membrane forms a soft skirt or the like all along the edges of the rear opening of the cushion 20. A unique membrane is preferably used; however, in alternative embodiments, several membranes may be superimposed.

Preferably, the rear outer surface of the cushion 20 that comprises the peripheral edge regions surrounding the rear breathing opening, has a curved shape for better matching some nasal regions, in particular the two curved alae (CA) situated on both sides of the nostrils of the user P.

In the embodiment of Fig. 1, the mask body 10 comprises a front wall that is curved and/or flat, that is traversed by the front opening 11 that can have a circular shape or section and/or an inner diameter of about between 1 and 3 cm, for instance of between 15 and 25 mm. Preferably, the front wall of the mask body 10 has a thickness of between 2 and 6 mm.

The mask body 10 and the two lateral arms 14 are made of plastic material and molded in one piece, for instance by injection molding or any other suitable molding process. Typically, the plastic or polymer material can be PP, PC, PBT or Nylon^{®}, or similar materials.

Furthermore, as shown in Fig. 1, the mask body 10 further comprises several venting orifices 16, i.e. traversing holes, slots or similar, traversing the front wall and arranged around the front opening 11. They are configured for venting to the atmosphere at least a part of the CO₂-containing gaseous flow expired by the user P during his/her expiratory phases. The venting orifices 16 can be arranged in groups or arrays comprising several venting orifices 16. The group of venting orifices 16 can sandwich the front opening 11 of the mask body 10, i.e. arranged to the left and to right of the front opening 11. The venting orifices 16 are in fluid communication with the inner breathing chamber of the flexible cushion 20. The venting orifices 16 can have a cylindrical, a tronconical or any other suitable shape.

Furthermore, the rear breathing opening has a (quasi-) rectangular or similar (e.g. trapezoidal shape). It is arranged at the center of the curved rear surface including the peripheral edge regions surrounding the rear breathing opening of the supple rear part 21 of the flexible nasal cushion 20. A bridge element, such as a little band or the like, dividing the rear breathing opening, namely forming a bridge between the upper and lower edges of the rear breathing opening, i.e. in the supple rear part 21. The bridge element can have an arch-shape, for instance a "U" or "V"-shape, that slightly projects into the inner breathing chamber of the cushion 20. When the nasal mask 1 is donned by the patient P as shown in Fig. 3, the bridge element comes into contact with the outer region of the nose of the patient separating the nostrils, namely the nasal columella, so as to constitute a support or the like prohibiting a collapse, sagging or similar of the thin regions of the supple rear part 21 of the cushion 20. The bridge element is preferably molded in one-piece with the rest of the flexible cushion 20.

The cushion 20 can exist in several sizes, such as small (S), medium (M) and/or large (L) sizes, so that it can fit various morphologies of patients.

Other embodiments of MCM-type masks 1 useable with the headgear structure 30 of the present invention, as below detailed, are disclosed by EP-A-4279108 and EP-A-4249024.

As shown in Fig. 2 & 3, when donned by the patient P, the mask 1 further comprises a headgear structure 30, also called a headgear 30, comprising flexible straps or similar structures.

The headgear 30 is attached to headgear-connecting structures 15, such as slots, hooks or the like, arranged at the free end of the two arms 24. The headgear 30 is used for correctly positioning, holding and/or securing the mask 1 on the head of the patient P. In other words, the headgear 30 can be adjusted to pull the mask 1 against the face/nose with sufficient force to achieve a gas tight seal between the mask 1 and the wearer's nasal regions.

Typically, the headgear 30 comprises several elongated strap elements 31 used for maintaining and securing the mask 1 in a desired position on the head of the patient P. Preferably, the straps 31 are made of polymer or fabric materials that can be slightly elastic or the like.

In the frame of the present invention, the comfort of use for the patient of the mask 1 has been improved as below explained.

Thus, it has been noticed by the inventors of the present invention, in the frame of various empirical tests made with existing masks, that the discomfort that is often related to gas leaks, especially overnight, that can be due to a poor positioning of the mask of the patient's face and/or a poor tightness of the cushion or the like.

In other words, the discomfort and/or leaks are closely related to the relative positioning of the mask 1 to the headgear 30 and/or to tension forces exerted by the flexible/elastic strap elements 31 of the headgear 30 that are not sufficient so that the cushion 20 tends to collapse under its own weight until touching the upper lip of the patient and putting a pressure on it.

Thus, Fig. 2 shows the nasal mask of Fig. 1 equipped with a headgear structure 30 according to the prior art that may lead to discomfort and/or leaks.

The headgear 30 comprises several flexible elongated strap elements 131 attached together and forming, when donned by the patient P, a tridimensional structure arranged on the patient's head.

The several flexible elongated strap elements 131 comprise :
- two lateral straps 132 each comprising a proximal end 132a comprising fixation means 136 configured to be attachable to headgear-connecting structures 15 of the two lateral arms 14 of the respiratory mask 1, and a distal end 132b;
- an over-head strap 133 forming an arch over the top of the head of the patient P, and
- a rear-head strap 134 passing around the back head of the patient P.

Those straps 132, 133, 134 meet and are attached together in two liaison regions 135 located on the right and left sides of the head.

As one can see in Fig. 2, the axis XX of the over-head strap 133 and the axis YY of the rear-head strap 134 define together, in the liaison region 135, an angle A of more than 90°, i.e. of about 100°.

It has been noticed by the inventors of the present invention that such an arrangement according to the prior art may lead to leaks and/or discomfort of the patient P, due to insufficient tension forces provided by the over-head strap 133 and/or the rear-head strap 134, especially the over-head strap 133, leading to a poor positioning of the mask 1 and/or a poor tightness of the cushion 20.

According to the present invention, to overcome this problem, the inventors have modified the headgear structure 30 as shown in Fig. 3 to 5.

The headgear structure 30 comprises, as the headgear 30 of Fig. 2, several flexible elongated strap elements 31 comprising two lateral strap elements 32 each comprising a proximal end 32a comprising fixation means 36 configured to be attachable to the headgear-connecting structures 15, such as hooks, slots, holes or the like, of the respiratory mask 1, and further a distal end 32b.

The fixation means 36 are arranged at the proximal ends 32a of the two lateral strap elements 32 can comprise Velcro^{®}-type structures and are configured for being foldable for forming loops or the like thereby allowing attaching the headgear 30 to the headgear connecting structures 15 of the mask body 10. However, in other embodiments, other types of fixation means 36 are possible.

Typically, a Velcro^{®}-like structure comprises a little-hook region configured for being attached to a little-loop region that are arranged on (one or) each lateral strap element(s), when the free proximal end of the lateral strap elements 32 is folded over, thereby forming a fixation loop that can be attached to the headgear-connecting structures 15 of the mask body 1, such as hooks, slots or the like, as shown in Fig. 3.

Further, at least one of the two lateral strap elements 32 can also comprise markings arranged on the outer surface of said lateral strap element(s) 32, representing different tightening levels or forces of the straps 32.

Embodiments of Velcro^{®}-type structures and markings are disclosed by EP-A-3981455.

However, as shown in Fig. 3, the headgear structure 30 according to the present invention further comprises an annular-shape strap structure 37 comprising a first curved strap element 33 and a second curved strap element 34.

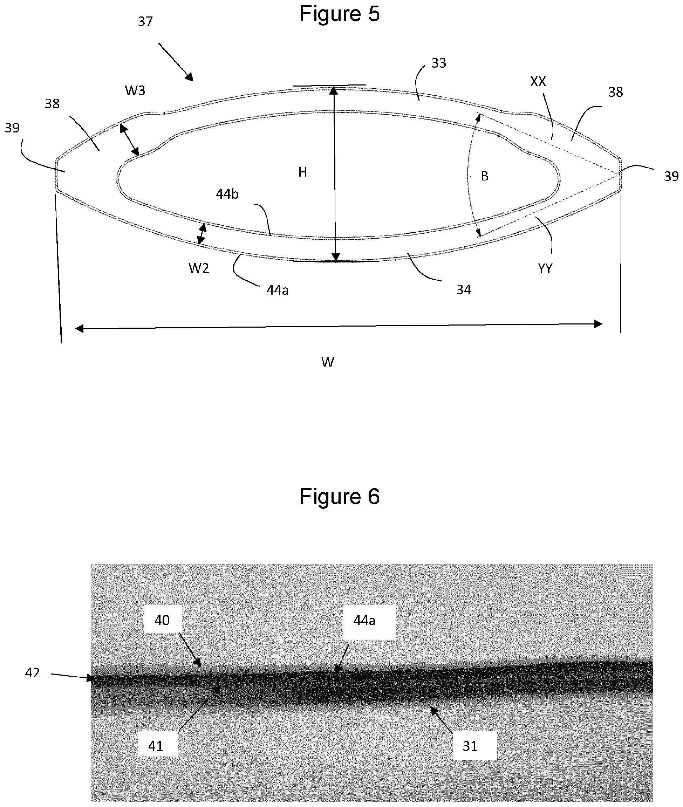

The annular-shape strap structure 37 has a generally oval-shape, namely an oval, ellipsoidal or similar shape as shown in Fig. 4 & 5.

Preferably, the annular-shape strap structure 37 has an elongated shape having a width W much larger than its height H, preferably W ≥ 2 . H, preferably W ≥ 2,5 . H, more preferably W ≥ 3 . H

For instance, when the headgear 30 is flat, as shown in Fig. 4 & 5, the width W of the annular-shape strap structure 37 can be of about 27,5 cm, and the height H of the annular-shape strap structure 37 is of about 8,4 cm. Further, the lateral strap elements 32 can have a first width W1 of about 1,2 cm, whereas the strap elements 33, 34 of the annular-shape strap structure 37 can have a second width (W2) also of about 1,2 cm. Of course, other dimensions are possible.

The first curved strap element 33 forms an upper or top half of the annular-shape strap structure 37, i.e. an upper strap element, whereas the second curved strap element 34 forms an lower or bottom half of the annular-shape strap structure 37, i.e. a lower strap element, as shown in Fig. 4 & 5.

As shown, the first curved strap element 33, i.e. the upper strap element, is curved upwardly or bent, i.e. toward the top of the head of the user P, when donned, whereas the second curved strap element 34, i.e. the lower strap element, is curved or bent downwardly, i.e. toward the neck of the head of the user P, when donned.

Further, the annular-shape strap structure 37, i.e. oval-shape structure, comprises two opposite end regions 39, preferably two diametrally-opposite end regions 39, and the distal ends 32b of the two lateral strap elements 32 are firmly attached to said opposite end regions 39. They are attached by Radio Frequency (RF) welding or any other suitable process.

Preferably, the first curved strap element 33, i.e. the upper strap element, comprises enlarged portions or zones 38 located in the two opposite end regions 39. For instance, said enlarged zones have a third width W3 of about 2 cm, i.e. with W3 > W2 (i.e. when the headgear 30 is flat as in Fig. 4 & 5).

Those enlarged zones 38 improve the comfort for the patient P, in use.

As one can see in Fig. 3 & 5, the axis XX of the first curved strap element 33 and the axis YY of the second curved strap element 34 define together, in the two opposite end regions 39, an angle B that is much less that the angle A of the headgear of Fig. 2, i.e. an angle B of less than 90°, typically of about 40° to 60°, preferably of between 45° and 50°.

The angle B is measured when the headgear structure 30 is deployed and/or is lying flat on a surface, as represented in Fig. 4 or 5.

Such an angle configuration participates in improving the comfort provided by the headgear 30, when donned, thanks to tension forces better oriented, resulting in a better positioning and maintaining of the mask 1 on the patient's head and further a better tightness of the cushion 20, i.e. reduction of gas leaks.

The annular-shape strap structure 37 having the generally oval-shape is preferably a one-piece structure as shown in Fig. 5. However, according to other embodiments, it can be made of several pieces or sub-units attached together.

Further, the two lateral strap elements 32 and the annular-shape strap structure 37 have a multi-layer structure 40, 41, 42 comprising several superimposed layers, as shown in Fig. 6.

Preferably, the multi-layer structure 40- 42 can comprise :
- a first fabric layer 40 comprising a first polymer material,
- a second fabric layer 42 comprising a second polymer material, and
- an intermediate foam layer 41 sandwiched between the first fabric layer 40 and the second fabric layer 42.

The second polymer material can be identical to or different from the first polymer material, for instance both are made of a polyamide material, such as Nylon ^{®}. Further, the intermediate foam layer 41 comprises for instance polyurethane material.

The first fabric layer 40, the second fabric layer 42 and the intermediate foam layer 41 are bonded together by flame lamination.

Preferably, the opposite edges 44, 45 of the multi-layer structure 40-42 are sealed by flame lamination.

More preferably, at least a portion of the first fabric layer 40 can comprise, constitute or exhibit a fluffy surface/coating that is oriented toward the exterior. This fluffy surface/coating can constitute the little-loop region of the Velcro^{®}-like structure that constitutes a good grip, for the little hooks, thereby facilitating the attachment of the folded part of the lateral strap elements 32 that is folded over for forming the fixation loop that is attached to the headgear-connecting structures 15 of the mask body 1, as shown in Fig. 3.

Further, the second fabric layer 42 preferably can comprise, constitute or exhibit a soft surface or coating that limits or avoids entanglements with the patient's hair, especially long hair, for instance when the patient sets up the headgear 30 on his/her head. The second fabric layer 42 is oriented toward the skin of the patient, while the headgear is donned/used.

The headgear 30 of the invention allows generating a leverage effect or similar due to the tension of the flexible/elastic traps 31 exerted on the two arms 24 of the mask body 10, which tends to pull up the cushion 20, in particular the regions at the bottom of the cushion 20, thereby improving the gas tightness and avoiding or limiting the gas leaks responsible for the discomfort of the patient P donning the mask 1, while preventing that the cushion 20 collapses toward the patient's upper lip.

Further, the annular-shape strap structure 37 of the headgear 30 of the invention also avoids that the headgear 30 glides along the back part of the head of the user as shown in Fig. 3 (i.e. arrow 12), which can be the case with prior art headgear configurations.

In other words, thanks to the new design of the headgear 30, it is possible to maintain the cushion 20 in a "raised" position that suppresses and/or limits the gas leaks, which is very important as prevention of air leaks is one of the most important features that a non-invasive ventilation (NIV) mask must fulfill.

The nasal mask 1 equipped with a headgear 30 according to the present invention can be used in a method for treatment of a respiratory disorder or condition affecting infant, toddler, child or adult patients, such as OSA or other respiratory troubles.

## Claims

1. Headgear structure (30) for a respiratory mask (1), comprising several flexible elongated strap elements (31) comprising two lateral strap elements (32) each comprising a proximal end (32a) comprising fixation means (36) configured to be attachable to headgear-connecting structures (15) of the respiratory mask (1), and further a distal end (32b), **characterized in that** it further comprises an annular-shape strap structure (37) comprising a first curved strap element (33) and a second curved strap element (34), wherein the distal ends (32b) of the two lateral strap elements (32) are firmly attached to the annular-shape strap structure (37).

2. Headgear structure according to Claim 1, **characterized in that** the annular-shape strap structure (37) has an elongated shape with a width (W) and a height (H) so that : W ≥ 2.H, preferably W ≥ 2,5.H, more preferably W ≥ 3.H.

3. Headgear structure according to any one of the preceding Claims, **characterized in that** the annular-shape strap structure (37) comprises two opposite end regions (39), and the distal ends (32b) of the two lateral strap elements (32) are attached to said opposite end regions (39), preferably two diametrally-opposite end regions (39).

4. Headgear structure according to any one of the preceding Claims, **characterized in that** the annular-shape strap structure (37) has a generally oval-shape, preferably an oval or an ellipsoidal shape.

5. Headgear structure according to any one of the preceding Claims, **characterized in that** two lateral strap elements (32) and the annular-shape strap structure (37) have a multi-layer structure (40, 41, 42) comprising :
- a first fabric layer (40) comprising at least a first polymer material,
- a second fabric layer (42) comprising at least a second polymer material, and
- an intermediate foam layer (41) sandwiched between the first fabric layer (40) and the second fabric layer (42).

6. Headgear structure according to Claim 5, **characterized in that** the first polymer material and/or the second polymer material are chosen among polyamide materials, such as Nylon ^{®}.

7. Headgear structure according to Claim 5, **characterized in that** the intermediate foam layer (41) comprises polyurethane material.

8. Headgear structure according to Claim 5, **characterized in that** the first fabric layer (40), the second fabric layer (42) and the intermediate foam layer (41) are bonded together by flame lamination.

9. Headgear structure according to Claim 5, **characterized in that** the opposite edges (44, 45) of the multi-layer structure (40, 41, 42) comprising the first fabric layer (40), the second fabric layer (42) and the intermediate foam layer (41), are sealed.

10. Headgear structure according to Claim 1, **characterized in that** the fixation means (36) arranged at the proximal ends (32a) of the two lateral strap elements (32) comprise Velcro^{®}-type structures.

11. Headgear structure according to Claim 3, **characterized in that** the opposite end regions (39) of the annular-shape strap structure comprise each an enlarged zone (38), and the distal ends (32b) of the two lateral strap elements (32) are attached to said enlarged zones (38).

12. Headgear structure according to Claim 3, **characterized in that** at least one of the flexible elongated strap elements (31) is stretchable.

13. Respiratory nasal mask (1) comprising:
- a mask body (10) comprising a front opening (11) and two elongated arms (14) attached to opposite lateral sides (11a, 11b) of the mask body (10), said elongated arms (14) comprising headgear-connecting structures (15),
- a hollow flexible cushion (20) comprising a supple rear part (21) comprising a rear breathing opening for allowing, in use, gas exchanges with nostrils of a user (P), said hollow flexible cushion (20) being detachably attached to the mask body (10), and
- a headgear structure (30) according to any one of the preceding Claims, attached to at least one of the headgear-connecting structures (15) of the two elongated arms (14).

14. Respiratory nasal mask according to Claim 13, **characterized in that** the supple rear part (21) of the flexible cushion (20) comprises an upper lip area (ULA) located immediately under the nostrils of the patient (P), two curved alae (CA) on both sides of the nostrils of the patient (P) and a tip region (TR) of the nose of the patient (P), when the mask (1) is donned by the patient (P).

15. Installation for providing gas to a patient (P) comprising a medical ventilator, a respiratory nasal mask (1) according to any one of Claims 13 or 14, equipped with a headgear structure (30) according to any one of Claims 1 to 13 attached to the elongated arms (14) of the respiratory nasal mask (1), said medical ventilator being fluidly and mechanically connected to the respiratory nasal mask (1) by means of a flexible hose (50).

## Amended claims

### Amended claims in accordance with Rule 137(2) EPC.

1. Headgear structure (30) for a respiratory mask (1), comprising several flexible elongated strap elements (31) comprising two lateral strap elements (32) each comprising a proximal end (32a) comprising fixation means (36) configured to be attachable to headgear-connecting structures (15) of the respiratory mask (1), and further a distal end (32b), and further comprising an annular-shape strap structure (37) comprising a first curved strap element (33) and a second curved strap element (34), wherein the distal ends (32b) of the two lateral strap elements (32) are firmly attached to the annular-shape strap structure (37),
**characterized in that** the two lateral strap elements (32) and the annular-shape strap structure (37) have a multi-layer structure (40, 41, 42) comprising :
- a first fabric layer (40) comprising at least a first polymer material chosen among polyamide materials,
- a second fabric layer (42) comprising at least a second polymer material chosen among polyamide materials, and
- an intermediate foam layer (41) of polyurethane material sandwiched between the first fabric layer (40) and the second fabric layer (42),
and wherein :
- the first fabric layer (40), the second fabric layer (42) and the intermediate foam layer (41) are bonded together by flame lamination, and
- the opposite edges (44, 45) of the multi-layer structure (40, 41, 42) comprising the first fabric layer (40), the second fabric layer (42) and the intermediate foam layer (41), are sealed.

2. Headgear structure according to Claim 1, **characterized in that** the annular-shape strap structure (37) has an elongated shape with a width (W) and a height (H) so that : W ≥ 2.H, preferably W ≥ 2,5.H, more preferably W ≥ 3.H.

3. Headgear structure according to any one of the preceding Claims, **characterized in that** the annular-shape strap structure (37) comprises two opposite end regions (39), and the distal ends (32b) of the two lateral strap elements (32) are attached to said opposite end regions (39), preferably two diametrally-opposite end regions (39).

4. Headgear structure according to any one of the preceding Claims, **characterized in that** the annular-shape strap structure (37) has a generally oval-shape.

5. Headgear structure according to Claim 4, **characterized in that** the annular-shape strap structure (37) has an oval or an ellipsoidal shape.

6. Headgear structure according to Claim 1, **characterized in that** the first polymer material and/or the second polymer material are Nylon ^{®}.

7. Headgear structure according to Claim 1, **characterized in that** at least a portion of the first fabric layer (40) comprises or exhibits a fluffy surface or coating that is oriented toward the exterior.

8. Headgear structure according to Claim 1, **characterized in that** the second fabric layer (42) comprises or exhibits a soft surface or coating, said second fabric layer (42) being oriented toward the skin of the patient, while the headgear is donned.

9. Headgear structure according to Claim 5, **characterized in that** the opposite edges (44, 45) of the multi-layer structure (40, 41, 42) are sealed by flame lamination.

10. Headgear structure according to Claim 1, **characterized in that** the fixation means (36) arranged at the proximal ends (32a) of the two lateral strap elements (32) comprise Velcro^{®}-type structures.

11. Headgear structure according to Claim 3, **characterized in that** the opposite end regions (39) of the annular-shape strap structure comprise each an enlarged zone (38), and the distal ends (32b) of the two lateral strap elements (32) are attached to said enlarged zones (38).

12. Headgear structure according to Claim 3, **characterized in that** at least one of the flexible elongated strap elements (31) is stretchable.

13. Respiratory nasal mask (1) comprising:
- a mask body (10) comprising a front opening (11) and two elongated arms (14) attached to opposite lateral sides (11a, 11b) of the mask body (10), said elongated arms (14) comprising headgear-connecting structures (15),
- a hollow flexible cushion (20) comprising a supple rear part (21) comprising a rear breathing opening for allowing, in use, gas exchanges with nostrils of a user (P), said hollow flexible cushion (20) being detachably attached to the mask body (10), and
- a headgear structure (30) according to any one of the preceding Claims, attached to at least one of the headgear-connecting structures (15) of the two elongated arms (14).

14. Respiratory nasal mask according to Claim 13, **characterized in that** the supple rear part (21) of the flexible cushion (20) comprises an upper lip area (ULA) located immediately under the nostrils of the patient (P), two curved alae (CA) on both sides of the nostrils of the patient (P) and a tip region (TR) of the nose of the patient (P), when the mask (1) is donned by the patient (P).

15. Installation for providing gas to a patient (P) comprising a medical ventilator, a respiratory nasal mask (1) according to any one of Claims 13 or 14, equipped with a headgear structure (30) according to any one of Claims 1 to 13 attached to the elongated arms (14) of the respiratory nasal mask (1), said medical ventilator being fluidly and mechanically connected to the respiratory nasal mask (1) by means of a flexible hose (50).
